# EUROPEAN PATENT APPLICATION

(11) **EP 0 911 000 A1**
(43) Date of publication of application: **28.04.1999**
(21) Application number: 98109105.1
(22) Date of filing: 19.05.1998
(51) Int. Cl.: A61F 5/48, A61F 13/42

(54) **System for monitoring the emission of organic fluids by an incontinent person**

(30) Priority: 23.10.1997 IT TO970926
(71) Applicant: Tacchino, Nicoletta, 15078 Rocca Grimalda (IT)
(72) Inventor: Tacchino, Nicoletta, 15078 Rocca Grimalda (IT)
(74) Representative: Cerbaro, Elena

(57) **Abstract**

A system (1) for monitoring the emission of organic fluids (2) by an incontinent person (3), wherein a remote control and signalling device (4) carried by a wearable absorbent retaining element (6) has a humidity sensor (7) and a transmitter (17) cooperating with the sensor (7) to emit an alarm signal (A(t)) when a humidity level measured inside the absorbent retaining element (6) is exceeded. The system (1) also has a central monitoring unit (5) with a receiver (23) for receiving the alarm signal (A(t)) and, on receiving the alarm signal (A(t)), activating indicators (28, 30) to indicate a condition of discomfort or risk to the person (3) fitted with the remote device (4).

## Description

The present invention relates to a system for monitoring the emission of organic fluids by an incontinent person.

The present invention may be used to advantage in institutions (e.g. hospitals, geriatric institutions, etc.) providing care for incontinent patients (such as babies or elderly people). As is known, patients in such institutions are provided with an absorbent retaining element (commonly known as a "sanitary towel" or "diaper") for retaining the organic fluids (normally urine) emitted by the patient; and prolonged contact of organic fluids with the patient's skin, or merely a prolonged high degree of humidity inside the absorbent retaining element, may cause skin rashes or even infections, the consequences of which are far from negligible in terms of both discomfort and treatment. This is particularly to be avoided in institutions caring for a large number of incontinent patients, in view of the irregularity of the emissions, staff shortages, and the many other duties the staff in such institutions are called upon to provide.

As such, assistance is invariably given some time after emission, thus resulting in discomfort to the patient and the hazards mentioned previously. Moreover, at times, the patient may not even be aware of the discomfort or hazard caused by the emission (often involuntary and uncontrolled) of such organic fluids, and therefore fails to request assistance.

It is an object of the present invention to overcome the aforementioned drawbacks by providing a system for automatically monitoring the emission of organic fluids by an incontinent person.

According to the present invention, there is provided a system for monitoring the emission of organic fluids by at least one incontinent person, characterized by comprising:
- at least one remote device for controlling and signalling said emission, and connectable to an absorbent retaining element worn by said person; and
- at least one central monitoring unit cooperating with said remote device;
   said remote device comprising:
- detecting means for generating an output signal correlated to the quantity of fluid in said absorbent retaining element; and
- transmission means receiving the output signal generated by said detecting means, and for generating in response an alarm signal;
   said central monitoring unit comprising:
- receiving means for receiving said alarm signal; and
- indicating means activated by said receiving means upon detection of an alarm signal indicating detection of a predetermined quantity of fluid in said absorbent retaining element.

The present invention also relates to a device for controlling and signalling the emission of organic fluids by an incontinent person.

According to the present invention, there is also provided a device for controlling and signalling the emission of organic fluids by an incontinent person, characterized by comprising:
- detecting means carried by an absorbent retaining element worn by said person, and for generating an output signal correlated to the quantity of fluid in said absorbent retaining element; and
- transmission means receiving the output signal generated by said detecting means, and for generating in response an alarm signal.

The present invention also relates to an absorbent retaining element for retaining organic fluids emitted by an incontinent person.

According to the present invention, there is also provided an absorbent retaining element for retaining organic fluids emitted by an incontinent person, characterized by comprising at least one device for controlling and signalling the emission of organic fluids into the absorbent retaining element; said device comprising:
- detecting means located in a given region of the absorbent retaining element to generate an output signal correlated to the quantity of organic fluid in the absorbent retaining element; and
- transmission means receiving the output signal generated by said detecting means, and for generating in response an alarm signal.

A non-limiting embodiment of the present invention will be described by way of example with reference to the accompanying drawings, in which:
Figure 1 shows, schematically, an absorbent retaining element in accordance with the teachings of the present invention;
Figure 2 shows, schematically, the electric circuits defining the monitoring system according to the teachings of the present invention;
Figure 3 shows a schematic view of the Figure 2 monitoring system.

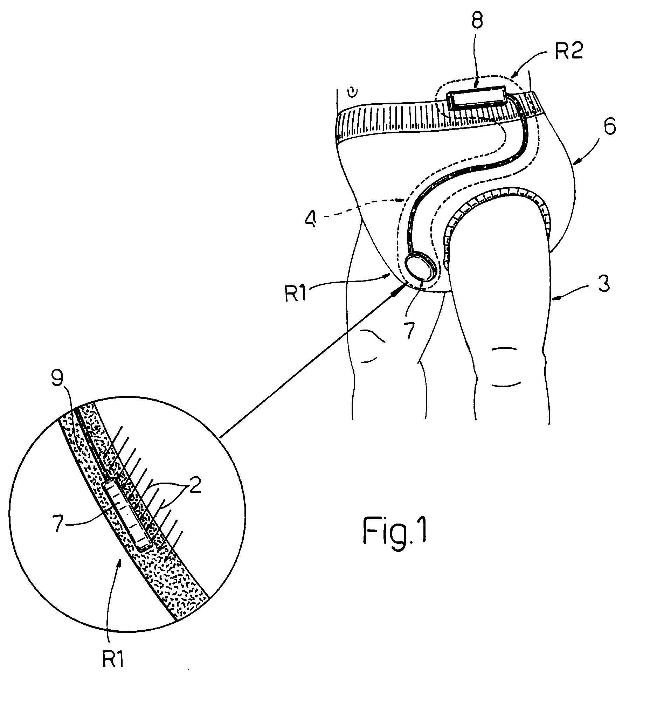

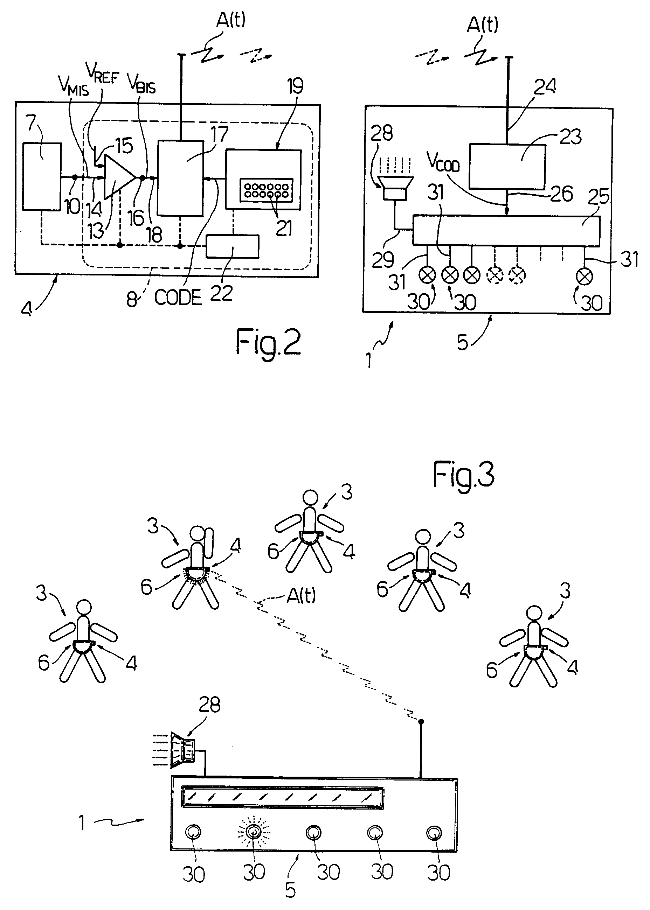

With reference to Figures 1 and 2, number 1 indicates as a whole a system for monitoring the emission of organic fluids 2 by an incontinent patient 3, and which comprises at least one remote control and signalling device 4 for controlling and signalling the emission of fluids 2, and at least one central monitoring unit 5 for receiving an alarm signal A(t) from device 4 and monitoring any discomfort or hazard to patient 3. More specifically, device 4 is carried by an absorbent retaining element 6 (commonly known as a "sanitary towel" or "diaper") worn about the pelvic region of patient 3.

Control and signalling device 4 comprises a known humidity sensor 7 located in a central region R1 of element 6 to detect the degree of humidity inside element 6; and a processing and transmission device 8 located in an end region R2 of element 6 and cooperating with sensor 7 to emit alarm signal A(t).

More specifically, sensor 7 is located inside the absorbent fabrics 9 of element 6 to prevent direct contact with the skin of patient 3, and comprises an output terminal 10 (Figure 2) at which is present an electric signal Vmis (in particular, a voltage proportional to the degree of humidity detected) correlated to the quantity of fluid 2 in absorbent fabrics 9 surrounding sensor 7.

Processing and transmission device 8 comprises a known comparator 13 having a first input terminal 14 connected to terminal 10 and receiving signal Vmis, and a second input terminal 15 receiving a reference signal Vref.

Comparator 13 compares signal Vmis with signal Vref to generate, at a respective output terminal 16, a bistable signal Vbis, which has a first state indicating element 6 is substantially dry, when signal Vmis is lower than signal Vref, and a second state indicating absorption of a significant quantity of fluid 2 by element 6, when signal Vmis is higher than signal Vref.

Device 8 also comprises a known radiotransmitter 17 having an enabling input 18 connected to terminal 16 to receive bistable signal Vbis. Radiotransmitter 17 remains on standby as long as bistable signal Vbis remains in the first state (low degree of humidity detected inside element 6), and automatically transmits alarm signal A(t) when bistable signal Vbis assumes the second state (high degree of humidity detected inside element 6).

Radiotransmitter 17 is also connected to a coding circuit 19 forming part of device 8, and which supplies radiotransmitter 17 with a CODE defining an identifier of control and signalling device 4. Radiotransmitter 17 processes (in known manner) the CODE from coding circuit 19 to generate and transmit an alarm signal A(t) containing information identifying device 4. System 1 in fact preferably operates with a number of remote devices 4 (Figure 3), each carried by a respective patient and having a respective identification CODE, so that each alarm signal A(t) from a respective remote device 4 represents the patient 3 assigned device 4 and, more specifically, a condition of discomfort or risk to patient 3.

Coding circuit 19 of each device 8 comprises a number of selectively activated microswitches 21 for altering the respective CODE in known manner; and each device 8 comprises a supply battery 22 connected to all the components of respective device 4.

Central monitoring unit 5 (Figure 2) comprises a known radioreceiver 23 with an input 24 (e.g. connected to a known aerial device not shown) to receive alarm signals A(t) from respective remote devices 4; and a decoding circuit 25 connected at the input to an output 26 of radioreceiver 23 to receive a coded electric signal Vcod containing the same information as the received alarm signal A(t). Decoding circuit 25 decodes coded signal Vcod to determine emission of an alarm signal A(t) and the device 4 by which alarm signal A(t) was emitted.

Unit 5 also comprises a known acoustic indicator 28 connected to an output 29 of decoding circuit 25, and which is activated when an alarm signal A(t) is received. Finally, unit 5 also comprises a number of known optical indicators 30, each of which is associated with a respective device 4, is connected to a respective output 31 of decoding circuit 25, and is activated upon radioreceiver 23 receiving alarm signal A(t) from device 4, i.e. in the event signal Vcod contains the CODE of the respective device 4.

In actual use, when sensor 7 of a device 4 detects a high degree of humidity inside respective absorbent retaining element 6, signal Vmis is higher than signal Vref and radiotransmitter 17 is enabled to transmit respective coded alarm signal A(t) to unit 5.

On receiving alarm signal A(t), radioreceiver 23 activates acoustic indicator 28 by means of decoding circuit 25 to indicate, fully automatically, a condition of discomfort or risk to at least one patient 3.

As stated, radioreceiver 23 receives alarm signal A(t) containing the identification CODE, and supplies decoding circuit 25 with signal Vcod also containing the CODE; decoding circuit 25 then decodes signal Vcod and activates optical indicator 30 associated with the CODE, to also indicate the patient 3 to which the condition of discomfort or risk applies.

In a variation not shown, the system for monitoring the emission of organic fluids comprises a number of central monitoring units, each of which, for example, is a portable monitoring unit which may be carried by each member of staff responsible for assisting incontinent patients, to monitor the condition of all the patients at all times.

System 1 as described affords considerable advantages, by being economical, reliable, easily adapted to institutions caring for incontinent patients 3, and by enabling immediate assistance of patients 3. Acoustic indicator 28 and optical indicator 30 in fact enable patient 3 in a condition of discomfort or risk to be identified and assisted immediately, thus preventing the consequences mentioned previously.

## Claims

1. A system (1) for monitoring the emission of organic fluids (2) by at least one incontinent person (3), characterized by comprising:
- at least one remote device (4) for controlling and signalling said emission, and connectable to an absorbent retaining element (6) worn by said person (3); and
- at least one central monitoring unit (5) cooperating with said remote device (4);
said remote device (4) comprising:
- detecting means (7) for generating an output signal (Vmis) correlated to the quantity of fluid (2) in said absorbent retaining element (6); and
- transmission means (8, 17) receiving the output signal (Vmis) generated by said detecting means (7), and for generating in response an alarm signal (A(t));
said central monitoring unit (5) comprising:
- receiving means (23, 25) for receiving said alarm signal (A(t)); and
- indicating means (28, 30) activated by said receiving means (23, 25) upon detection of an alarm signal (A(t)) indicating detection of a predetermined quantity of fluid (2) in said absorbent retaining element (6).

2. A system as claimed in Claim 1, characterized in that said detecting means (7) comprise humidity sensing means (7) located inside said absorbent retaining element (6).

3. A system as claimed in Claim 1 or 2, characterized in that said remote device (4) comprises comparing means (13) cooperating with said detecting means (7) to emit an enabling signal (Vbis) for enabling said transmission means (8, 17) in the event of a predetermined relationship between said output signal (Vmis) and a given threshold value (Vref).

4. A system as claimed in any one of the foregoing Claims, characterized in that said indicating means (28, 30) comprise acoustic signalling means (28).

5. A system as claimed in any one of the foregoing Claims, characterized in that said indicating means (28, 30) comprise visual signalling means (30).

6. A system as claimed in any one of the foregoing Claims, characterized by comprising a number of remote devices (4) for controlling and signalling said emission, and each connectable to a respective absorbent retaining element (6) worn by a respective incontinent person (3); each said remote device (4) comprising respective coding means (19, 21) for forming an identification code (CODE) identifying the remote device (4);
each said remote device (4) associating the respective said identification code (CODE) with the respective said alarm signal (A(t)) to form a coded alarm signal (A(t));
said central monitoring unit (5) comprising decoding means (25) for decoding said coded alarm signal (A(t)), for extracting information associated with said identification code (CODE), and for indicating the remote device (4) by which the coded alarm signal (A(t)) was generated.

7. A system as claimed in Claim 6, characterized in that said coding means (19, 21) of each said remote device comprise respective selecting means (21) for forming a changeable identification code (CODE).

8. A device (4) for controlling and signalling the emission of organic fluids (2) by an incontinent person (3), characterized by comprising:
- detecting means (7) carried by an absorbent retaining element (6) worn by said person (3), and for generating an output signal (Vmis) correlated to the quantity of fluid (2) in said absorbent retaining element (6); and
- transmission means (8, 17) receiving the output signal (Vmis) generated by said detecting means (7), and for generating in response an alarm signal (A(t)).

9. A device as claimed in Claim 8, characterized in that said detecting means (7) comprise humidity sensing means (7) located in a given region (R1) of said absorbent retaining element (6) to detect the degree of humidity in said region (R1).

10. A device as claimed in Claim 8 or 9, characterized by comprising comparing means (13) cooperating with said detecting means (7) to emit an enabling signal (Vbis) for enabling said transmission means (8, 17) in the event of a predetermined relationship between said output signal (Vmis) and a given threshold value (Vref).

11. A device as claimed in any one of the foregoing Claims from 8 to 10, characterized by comprising coding means (19, 21) associated with said transmission means (8, 17) to form an identification code (CODE) identifying the device (4), and to code said alarm signal (A(t)).

12. A device as claimed in Claim 11, characterized in that said coding means (19, 21) comprise selecting means (21) for forming a changeable identification code (CODE).

13. An absorbent retaining element (6) for retaining organic fluids (2) emitted by an incontinent person (3), characterized by comprising at least one device (4) for controlling and signalling the emission of organic fluids into the absorbent retaining element (6); said device (4) comprising:
- detecting means (7) located in a given region (R1) of the absorbent retaining element (6) to generate an output signal (Vmis) correlated to the quantity of organic fluid (2) in the absorbent retaining element (6); and
- transmission means (8, 17) receiving the output signal (Vmis) generated by said detecting means (7), and for generating in response an alarm signal (A(t)).

14. An element as claimed in Claim 13, characterized in that said detecting means (7) comprise humidity sensing means (7) located in a given region (R1) of said absorbent retaining element (6) to detect the degree of humidity in said region (R1).

15. An element as claimed in Claim 13 or 14, characterized in that said device (4) comprises comparing means (13) cooperating with said detecting means (7) to emit an enabling signal (Vbis) for enabling said transmission means (8, 17) in the event of a predetermined relationship between said output signal (Vmis) and a given threshold value (Vref).

16. An element as claimed in any one of the foregoing Claims from 13 to 15, characterized in that said device (4) comprises coding means (19, 21) associated with said transmission means (8, 17) to form an identification code (CODE) identifying the device (4), and to code said alarm signal (A(t)).

17. An element as claimed in Claim 16, characterized in that said coding means (19, 21) comprise selecting means (21) for forming a changeable identification code (CODE).
